(19) Europäisches Patentamt / European Patent Office / Office européen des brevets

(11) EP 3 459 521 A1

(12) EUROPEAN PATENT APPLICATION
published in accordance with Art. 153(4) EPC

(43) Date of publication:
**27.03.2019 Bulletin 2019/13**

(21) Application number: **17824256.6**

(22) Date of filing: **04.07.2017**

(51) Int Cl.:
***A61K 8/64*** *(2006.01)* ***A61K 8/06*** *(2006.01)*
***A61K 8/898*** *(2006.01)* ***A61Q 19/00*** *(2006.01)*
***B01F 17/30*** *(2006.01)*

(86) International application number:
**PCT/JP2017/024552**

(87) International publication number:
**WO 2018/008653 (11.01.2018 Gazette 2018/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.07.2016 JP 2016133023**

(71) Applicant: **Kaneka Corporation**
**Osaka-shi, Osaka 530-8288 (JP)**

(72) Inventors:
- **TSUJI Tadao**
  **Settsu-shi**
  **Osaka 566-0072 (JP)**
- **MASUDA Toshiyuki**
  **Settsu-shi**
  **Osaka 566-0072 (JP)**
- **NOGUCHI Kisaburo**
  **Settsu-shi**
  **Osaka 566-0072 (JP)**

(74) Representative: **Hoffmann Eitle**
**Patent- und Rechtsanwälte PartmbB**
**Arabellastraße 30**
**81925 München (DE)**

# (54) EMULSIFIED COMPOSITION AND COSMETIC USING SAME

(57) The purpose of the present invention is to provide an emulsified composition that is reliably maintained in the emulsified state, and a cosmetic comprising the emulsified composition. This emulsified composition is characterized by comprising an amino-modified silicone oil, an aqueous solvent, and a surfactin represented by formula (I) below. In the formula, X represents an amino acid residue selected from the group consisting of a leucine residue, an isoleucine residue, and a valine residue, and $R^1$ represents a $C_{9-18}$ alkyl group. The concentration of the surfactin with respect to the entire emulsified composition is preferably 0.1-50 mM.

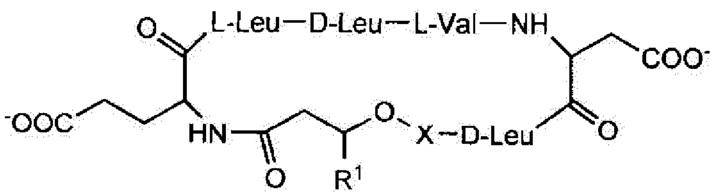

(I)

EP 3 459 521 A1

## Description

### TECHNICAL FIELD

The present invention relates to an emulsified composition having excellent emulsifying characteristics and a cosmetic containing the emulsified composition.

### BACKGROUND ART

Silicone has a structure in which mostly the side chains of polysiloxane are substituted with lower alkyl groups. By having excellent heat resistance or the like and also high safety to human body, and for exhibiting various property states such as an oil state, a rubber state, and resin state depending on molecular weight or the like, silicone is widely used in many fields. For example, as silicone oil exhibits an excellent lubricating property or water repellency, for the purpose of providing skin with fresh use feel by suppressing stickiness or for the purpose of providing hair with silky texture by enhancing the softness, silicone oil is blended with other oily components in a cosmetic.

However, silicone is chemically inactive and has low affinity for other compounds in some cases. As such, amino-modified silicone having an amino substituent introduced to a side chain is being developed. From the viewpoint that the amino-modified silicone is chemically active, the amino-modified silicone is used as an adhesive component or even for a coatable silicone sealant. Furthermore, from the viewpoint that the amino-modified silicone has an improved adhesion property and can provide flexibility or a lubricating property, the amino-modified silicone is also used as a component of a hair cosmetic for improving smoothness or preventing entanglement. Other than those, in view of the adsorption property or reactivity, the amino-modified silicone is also used for wax or the like.

Furthermore, the silicone oil has a problem that, by having low compatibility with oily components, it is difficult to form an emulsified system. As such, for a composition containing silicone oil, it is generally necessary to blend a surfactant in relatively large amount.

In Patent Document 1 and Patent Document 2, for example, a hair cosmetic containing biosurfactant and silicones is described, and, as one of specific examples of silicone, amino-modified silicone is used. Furthermore, in Patent Document 3, a thickened gel-like oily composition containing an anionic surfactant having a lipopeptide structure and an oily component is disclosed, and, as a specific example of the anion surfactant, a surfactin, which is a natural biosurfactant, is used.

Patent Document 1: Japanese Unexamined Patent Application, Publication No. 2011-26280
Patent Document 2: Japanese Unexamined Patent Application, Publication No. 2011-26281
Patent Document 3: Japanese Unexamined Patent Application, Publication No. 2003-176211

### DISCLOSURE OF THE INVENTION

#### Problems to be Solved by the Invention

As described above, a surfactant is generally used in a composition containing silicone oil.

However, there is a case in which such a composition cannot stably maintain the emulsified state even if the composition is in an emulsified state immediately after the production. As such, to maintain the emulsified state of a composition containing silicone oil, a method of increasing the blending amount of a surfactant, reducing the amount of silicone oil or oily components, or increasing the viscosity of an aqueous phase has been conventionally employed. However, this method has a problem that an increased irritating property is yielded or limitation occurs in constituting a composition.

In this regard, an object of the present invention is to provide an emulsified composition that is stably maintained in an emulsified state, and a cosmetic containing the emulsified composition.

#### Means for Solving the Problems

To solve the problems described above, the present inventors have repeated intensive studies. As a result, the present inventors have found that, for stably maintaining the emulsified state of a composition containing an amino-modified silicone oil, which is useful as a component of a hair cosmetic or the like, surfactin as one kind of biosurfactants is quite suitable, and completed the present invention accordingly.

Hereinbelow, the present invention is described.

[1] An emulsified composition containing an amino-modified silicone oil, an aqueous solvent, and a surfactin represented by the formula (I) below:

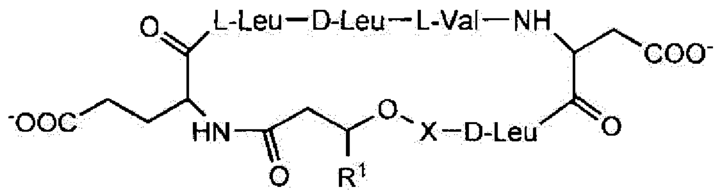

(I)

[in the formula,

X represents an amino acid residue selected from the group consisting of a leucine residue, an isoleucine residue, and a valine residue; and
$R^1$ represents a $C_{9-18}$ alkyl group].

[2] The emulsified composition described in above [1], further containing an oily component.
[3] The emulsified composition described in above [1] or [2], in which a concentration of the surfactin is 0.1 mM or more and 50 mM or less with respect to the entire emulsified composition.
[4] A cosmetic containing the emulsified composition described in any one of above [1] to [3].

Effects of the Invention

In the emulsified composition according to the present invention, the emulsified state is stably maintained at even less amount of a surfactant while the composition contains an amino-modified silicone oil. Furthermore, from the viewpoint that the surfactin as a surfactant to be blended in the emulsified composition according to the present invention is a natural peptide surfactant, the surfactin has low skin permeability, and thus considered to have low transdermal toxicity compared to synthetic surfactants that are universally used. Furthermore, even when released to an environment, the surfactin is rapidly degraded by microorganisms or the like, and thus has a low load given to an environment. Accordingly, the emulsified composition according to the present invention has safety and high product value, and thus can be suitably used for a cosmetic or the like.

BRIEF DESCRIPTION OF THE DRAWINGS

FIG. 1 is a graph showing the result of an experiment for testing the emulsified state-maintaining action of sodium surfactin on a composition containing water and various silicone oils.
FIG. 2 is a graph showing the result of an experiment for testing the emulsified state-maintaining action of sodium dodecyl sulfate on a composition containing water and various silicone oils.
FIG. 3 is a graph showing the result of an experiment for testing the emulsified state-maintaining action of low-concentration sodium surfactin on a composition containing water and various silicone oils.
FIG. 4 is a graph showing the result of an experiment for testing the emulsified state-maintaining action of low-concentration sodium dodecyl sulfate on a composition containing water and various silicone oils.
FIG. 5 is a graph showing the result of an experiment for testing the emulsified state-maintaining action of sodium surfactin or sodium decyl sulfate on a composition containing water and an amino-modified silicone oil.
FIG. 6 is a graph showing the result of an experiment for testing the emulsified state-maintaining action of low-concentration sodium surfactin or sodium decyl sulfate on a composition containing water and an amino-modified silicone oil.

PREFERRED MODE FOR CARRYING OUT THE INVENTION

An emulsified composition according to the present invention is characterized by containing an amino-modified silicone oil, an aqueous solvent, and a surfactin represented by the formula (I) above.
In general, the amino-modified silicone oil is a compound which is liquid at room temperature, in which part of the alkyl group in $C_{1-4}$ alkylpolysiloxane is substituted with an amino substituent. Examples of the amino substituent of the amino-modified silicone oil may include an amino group ($-NH_2$), a mono ($C_{1-4}$ alkyl) amino group, a di($C_{1-4}$ alkyl) amino group, an amino-$C_{1-4}$ alkylene group, a mono ($C_{1-4}$ alkyl) amino-$C_{1-4}$ alkylene group, a di($C_{1-4}$ alkyl) amino-$C_{1-4}$

alkylene group, an amino-$C_{1-4}$ alkylene-amino-$C_{1-4}$ alkylene group, a mono ($C_{1-4}$ alkyl) amino-$C_{1-4}$ alkylene-amino-$C_{1-4}$ alkylene group, and a di ($C_{1-4}$ alkyl) amino-$C_{1-4}$ alkylene-amino-$C_{1-4}$ alkylene group. Furthermore, as long as the substitution is given at the amino substituent, it can be substituted with other substituent. Examples of other substituent may include a $C_{1-4}$ alkyl-$C_{2-4}$ alkylene glycol residue-$C_{1-4}$ alkylene group. In a di ($C_{1-4}$ alkyl) amino group, an amino-$C_{1-4}$ alkylene-amino-$C_{1-4}$ alkylene group, or the like, plural $C_{1-4}$ alkyl groups may be the same as or different from each other, and plural $C_{1-4}$ alkylene groups may be the same as or different from each other. The same holds true for a $C_{2-4}$ alkylene glycol residue, and it may be either a polyethylene glycol residue or a polypropylene glycol residue, and it may be also an ethylene glycol-propylene glycol copolymer residue. Incidentally, in the present specification, the "glycol residue" indicates an atomic group as a remainder after excluding hydrogen atoms from two alcohol hydroxyl groups that are included in glycol, and the "glycol copolymer residue" indicates an atomic group as a remainder after excluding hydrogen atoms from two alcohol hydroxyl groups that are included in the glycol copolymer. Only one kind of the amino-modified silicone oil may be used, or two or more kinds of the amino-modified silicone oils may be used.

In the present invention, examples of the "$C_{1-4}$ alkyl group" may include a methyl group, an ethyl group, an n-propyl group, an isopropyl group, and an n-butyl group, a methyl group or an isopropyl group is preferable, and a methyl group is more preferable. Examples of the "$C_{1-4}$ alkylene group" may include a methylene group, an ethylene group, an n-propylene group, and an n-butylene group, and a $C_{1-2}$ alkylene group is preferable.

Molecular weight or polymerization degree of the amino-modified silicone oil may be suitably selected depending on desired viscosity or the like of the amino-modified silicone oil itself or emulsified composition. For example, it is possible to select an amino-modified silicone oil of which viscosity at 25°C is about 500 $mm^2/s$ or more and 10000 $mm^2/s$ or less. From the viewpoint of the obtainability of the amino-modified silicone oil, handling property of an emulsified composition to be obtained, or the like, the viscosity is preferably 500 $mm^2/s$ or more and 2000 $mm^2/s$ or less, more preferably 600 $mm^2/s$ or more and 1500 $mm^2/s$ or less, and even more preferably 700 $mm^2/s$ or more and 1000 $mm^2/s$ or less.

Blending amount of the amino-modified silicone oil is not particularly limited, and may be suitably adjusted. However, for example, the blending amount can be set to 0.1% by mass or more and 80% by mass or less with respect to the entire emulsified composition. As the ratio is 0.1% by mass or more, characteristics of the amino-modified silicone oil can be more certainly exhibited in the emulsified composition. Meanwhile, the ratio is preferably 80% by mass or less, since easy emulsification can be readily obtained if the ratio is not excessively high. The ratio is more preferably 0.5% by mass or more, and even more preferably 1.0% by mass or more. Furthermore, the ratio is more preferably 60% by mass or less or 50% by mass or less, and even more preferably 30% by mass or less or 20% by mass or less.

The aqueous solvent is not particularly limited as long as it is a solvent which has water as a main component, and examples thereof may include water and a mixed solvent of water and a water-miscible organic solvent. Ratio of water in the mixed solvent may be suitably adjusted, but for example, the ratio can be set to 60% by volume or more or 70% by volume or more, and is more preferably 80% by volume or more or 90% by volume or more, and even more preferably 95% by volume or more. Upper limit of the ratio of water in the mixed solvent is not particularly limited, and the upper limit may be less than 100% by volume, 99.5% by volume, or 99% by volume. Incidentally, in the present specification, the ratio expressed with "% by volume" indicates a value at 20°C.

The "water-miscible organic solvent" indicates an organic solvent which can be homogeneously mixed in an amount of 5 g or more in 100 mL of water at 20°C, for example. Examples of the water-miscible organic solvent may include an alcohol-based solvent such as methanol, ethanol, or isopropanol; an ether-based solvent such as diethyl ether or tetrahydrofuran; a ketone-based solvent such as acetone; a nitrile-based solvent such as acetonitrile; an amide-based solvent such as dimethyl formamide or dimethyl acetamide; a sulfoxide-based solvent such as dimethyl sulfoxide; and a carboxylic acid-based solvent such as formic acid and acetic acid. Suitably, a water-miscible organic solvent that is miscible with water at any ratio is used.

Blending amount of the aqueous solvent is not particularly limited, and may be suitably adjusted. However, the blending amount can be set to 50% by mass or more and 99% by mass or less with respect to the entire emulsified composition. As the ratio is 50% by mass or more, watersoluble components including surfactin (I) can be suitably dissolved. Meanwhile, the ratio is preferably 99% by mass or less, since easy emulsification can be readily obtained if the ratio is not excessively high. The ratio is more preferably 70% by mass or more, and even more preferably 80% by mass or more. Furthermore, the ratio is more preferably 95% by mass or less, and even more preferably 90% by mass or less.

The emulsified composition according to the present invention contains a surfactin that is represented by the following formula (I) (in the present invention, it may be abbreviated as "surfactin (I)"). In the emulsified composition according to the present invention, a dispersion state of the amino-modified silicone oil is significantly improved by blending of the surfactin (I), in particular, and thus it becomes possible to maintain stably the emulsified state. Furthermore, since the surfactin (I) is a peptide compound, the surfactin (I) has a small load applied to a natural environment, and thus is safe to a human body.

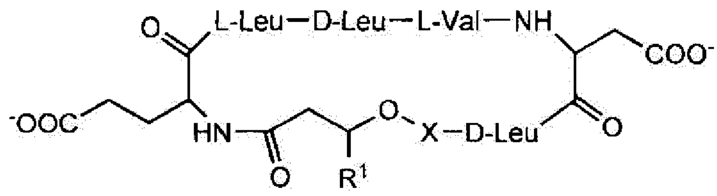

(I)

[in the formula,

X represents an amino acid residue selected from the group consisting of a leucine residue, an isoleucine residue, and a valine residue; and
$R^1$ represents a $C_{9-18}$ alkyl group]

The amino acid residue as X may be either L form or D form, but is preferably L form.

The "$C_{9-18}$ alkyl group" indicates a linear or branched monovalent saturated hydrocarbon group with carbon atom number of 9 or more and 18 or less. Examples thereof include an n-nonyl group, a 6-methyloctyl group, a 7-methyloctyl group, an n-decyl group, a 8-methylnonyl group, an n-undecyl group, a 9-methyldecyl group, an n-dodecyl group, a 10-methylundecyl group, an n-tridecyl group, a 11-methyldodecyl group, an n-tetradecyl group, an n-pentadecyl group, an n-hexadecyl group, an n-heptadecyl group, and an n-octadecyl group.

Only one kind of the surfactin (I) may be used, or two or more kinds of the surfactin (I) may be used. For example, plural surfactins (I) which have a different $C_{9-18}$ alkyl group represented by $R^1$ may be used.

The surfactin (I) can be isolated from a culture solution after culturing bacterial strain belonging to Bacillus subtilis according to a known method, for example, and the surfactin (I) may be an adjusted product or a purified product or may be used as a non-purified product, for example, as a culture solution itself. Furthermore, a surfactin obtained by a chemical synthesis method can be also used in the same manner.

As a raw material of the surfactin (I), a salt of the surfactin (I) may be used. The counter cation $M^+$, which constitutes a salt of the surfactin (I), is not particularly limited, but examples thereof include an alkali metal ion and a quaternary ammonium ion.

The alkali metal ion which can be used for a salt of the surfactin (I) is not particularly limited, but examples thereof include a lithium ion, a sodium ion, and a potassium ion. Furthermore, in a case in which two or more kinds of alkali metal ions are used, they may be the same as or different from each other.

Examples of a substituent of the quaternary ammonium ion include an organic group like a $C_{1-4}$ alkyl group such as a methyl group, an ethyl group, an n-propyl group, an isopropyl group, an n-butyl group, or a t-butyl group; a $C_{7-14}$ aralkyl group such as a benzyl group, a methylbenzyl group, or a phenylethyl group; and a $C_{6-12}$ aryl group such as a phenyl group, a toluyl group, or a xylyl group. Examples of the quaternary ammonium ion include a tetramethyl ammonium ion, a tetraethyl ammonium ion, and a pyridinium ion.

Incidentally, in a salt of the surfactin (I), the two counter ions may be the same as or different from each other. Furthermore, between two $-COO^-$ that are present in the surfactin (I), it is also possible that one is in a state of-COOH or $-COO^-$ and the other is in a state of $-COO^-M^+$.

Blending amount of the surfactin (I) is not particularly limited, and may be suitably adjusted. However, the concentration of the surfactin (I) can be set to 0.1 mM or more and 50 mM or less with respect to the entire emulsified composition, for example. As the concentration is 0.1 mM or more, the emulsifying action by the surfactin (I) can be more certainly exhibited. Meanwhile, the concentration is preferably 50 mM or less, since the effect by the surfactin (I) is hardly saturated if the ratio is not excessively high. The concentration is more preferably 0.5 mM or more, and even more preferably 1 mM or more. Furthermore, the concentration is more preferably 40 mM or less, and even more preferably 30 mM or less.

The emulsified composition according to the present invention may additionally contain an oily component. Since there is a case in which the emulsified composition according to the present invention has a more improved dispersion state of the amino-modified silicone oil according to blending of an oily component, maintaining stably the emulsified state is more easily achieved. The oily component used in the present invention is not particularly limited as long as it is not mixed with water at any ratio, and preferred examples thereof include hydrocarbons such as squalane, fluid paraffin, light fluid isoparaffin, ceresin, polyethylene powder, squalene, microcrystalline wax, Vaseline, fluid isoparaffin, polybutene, or mineral oil; waxes such as bee wax, carnauba wax, candelilla wax, jojoba oil, lanolin, or whale wax; oils and fats such as macadamia oil, olive oil, cotton seed oil, soy bean oil, avocado oil, rice bran oil, rice oil, rice germ oil, palm seed oil, castor oil, rosehip oil, evening primrose oil, camellia oil, horse oil, grape seed oil, palm oil, meadow foam oil, shear butter, corn oil, safflower oil, or sesame oil; esters such as ethylhexyl palmitate, isononyl isononanoate, isopropyl myristate, ethyl oleate, glyceryl tri(caprylate·capreate), cetyl 2-ethylhexanoate, glyceryl tri 2-ethylhexanoate,

diisopropyl sebacate, or cholesteryl hydroxystearate; fatty acids such as myristic acid, stearic acid, or oleic acid; silicone oils such as methylpolysiloxane, methylphenylpolysiloxane, or amino-modified silicone; higher alcohols such as cetanol or oleyl alcohol; and alkyl glyceryl ethers such as batyl alcohol and chimyl alcohol. Only one kind of the oily component may be used, or two or more kinds of the oily component may be used.

In the present invention, the concentration of the oily component is preferably 0.5% by weight to 99% by weight, more preferably 0.88% by weight to 99% by weight, even more preferably 1% by weight to 98.88% by weight, and particularly preferably 10% by weight to 50% by weight. As the oily component is within the above concentration range, it becomes possible to form a stable emulsion.

The emulsified composition according to the present invention may be suitably blended with other addition components depending on the purpose of using the emulsified composition, or the like. Other addition components are not particularly limited, but examples thereof may include thickening polysaccharides such as guar gum and xanthan gum; celluloses such as hydroxypropyl cellulose and carboxymethyl cellulose; carboxyvinyl polymers such as an acrylic acid polymer or an acrylic acid copolymer; silicone compounds; coloring agents; pH adjusting agents; plant extracts; preservatives; chelating agents; vitamin preparations; pharmaceutical components such as an anti-inflammatory agent; fragrance; UV absorbing agents; and anti-oxidants. Furthermore, even in the emulsified composition according to the present invention or a product thereof, a conventional surfactant may be used in combination, in addition to the surfactin (I). However, it is preferable that all surfactants in the emulsified composition or product are the surfactin (I).

The product containing the emulsified composition according to the present invention is not particularly limited, and examples thereof may include those containing an amino-modified silicone oil like a cosmetic such as a cosmetic product for decoration, cream, gel, lotion, deodorant, or a UV blocking agent; a cosmetic product or a toiletry product such as shampoo, a shower bath product, an antiperspirant, a liquid toothpaste, or an oral cleanser; moisturized wipe such as a wet tissue which is used for cleansing cosmetics or wiping baby bottom; a sterilizing solution for sterilizing hands or the like for medical or household use; a fiber product; a rubber · plastic related product; a product of civil engineering · construction; a paper · pulp product; a mechanical · metal product; a cleaning product; a beverage or food product; a paint · ink product; a product for environment conservation; an agriculture · fertilizer product; a product for information industry; and other cleansing agents for industrial use.

A method for producing the emulsified composition according to the present invention is not particularly limited, and it is sufficient to have just mixing each component described above. However, to form an emulsified state, vigorous stirring by using a mixer, ultrasonic waves, or the like is preferable. The temperature at that time is sufficiently room temperature, and, specifically, may be set to about 10°C or higher and 50°C lower. The stirring time is not particularly limited, but may be set to about 10 seconds or longer and 1 hour or shorter, for example.

Since the emulsified composition according to the present invention has a stably maintained emulsified state mainly by the action of the surfactin (I), the emulsified composition is highly suitable for an application to a product containing an amino-modified silicone oil.

## EXAMPLES

Hereinbelow, the present invention will be described in more detail based on examples, but the present invention is not limited to those examples.

### Example 1

To a test tube, 2 g of silicone oil shown in Table 1 was added, 2 g of an aqueous solution of a surfactant, specifically, an aqueous solution of sodium surfactin or an aqueous solution of sodium dodecyl sulfate, was further added, and by mixing for 1 minute using a test tube mixer at room temperature, a mixture was obtained. Incidentally, the concentration of the aqueous solution of a surfactant was adjusted such that the final concentration is 25 mM. Furthermore, all of the silicone oils that have been used are products manufactured by Shin-Etsu Silicones.

[Table 1]

| Component name | Product name | Viscosity ($mm^2/s$) |
|---|---|---|
| Methylpolysiloxane | KF-96A-5cs | 5 |
| Methylpolysiloxane | KF-96A-20cs | 20 |
| Methylpolysiloxane | KF-96A-200cs | 200 |
| Methylpolysiloxane | KF-96A-500cs | 500 |
| Methylpolysiloxane | KF-96A-1000cs | 1000 |

(continued)

| Component name | Product name | Viscosity ($mm^2/s$) |
|---|---|---|
| Methylpolysiloxane | KF-96A-3000cs | 3000 |
| Methylpolysiloxane | KF-96A-5000cs | 5000 |
| Highly-polymerized methylpolysiloxane | KF-96H-10000cs | 10000 |
| Decamethylcyclopentasiloxane | KF-995 | 4 |
| Aminoethylaminopropylmethylsiloxane dimethylsiloxane copolymer | KF-8004 | 800 |

The mixture was left to stand still at 50°C, and the next day (i.e., 1 day later), 1 week later, 2 weeks later, and 3 weeks later, the whole height (total height of emulsified phase and aqueous phase) and the height of emulsified phase only were measured by using a height gauge. Then, an emulsification index was calculated based on the following formula.

$$\text{Emulsification index (\%)} = [(\text{Height of emulsified phase}) / (\text{Whole height})] \times 100$$

The result of using sodium surfactin is shown in FIG. 1 and the result of using sodium dodecyl sulfate is shown in FIG. 2. Incidentally, in FIG. 1, "SF" represents sodium surfactin, and, in FIG. 2, "SDS" represents sodium dodecyl sulfate.
Like the results shown in FIGs. 1 and 2, the emulsifying effect of sodium surfactin and sodium dodecyl sulfate hardly changes.
However, regarding the amino-modified silicone oil (product name of "KF-8004"), even if the emulsification can be initially achieved with sodium dodecyl sulfate, liquid separation is immediately yielded according to the still standing; on the other hand, it was evident that the emulsified state is relatively stably maintained when sodium surfactin is used. Accordingly, it was recognized that sodium surfactin tends to be suitable for emulsification by an amino-modified silicone oil.

Example 2

An experiment was carried out in the same manner as Example 1 above except that the final concentration of each surfactant was changed to 1/50, i.e., 0.5 mM. The result of using sodium surfactin is shown in FIG. 3 and the result of using sodium dodecyl sulfate is shown in FIG. 4.
Like the results shown in FIGs. 3 and 4, there is a tendency of generally having lower emulsifying effect in both of sodium surfactin and sodium dodecyl sulfate, and, in the case of sodium dodecyl sulfate, the emulsifying effect was not recognized at all depending on the type of the silicone oil.
In the case of the amino-modified silicone oil (product name of "KF-8004"), although the emulsifying effect was initially recognized even with sodium dodecyl sulfate, the emulsified state was not stable and separation was yielded over time.
On the other hand, in the case of blending sodium surfactin, a tendency of losing gradually the emulsified state was recognized, but the degree thereof was clearly lower compared to sodium dodecyl sulfate. As described above, even when the concentration of a surfactant is reduced, it was recognized that sodium surfactin tends to be suitable for emulsification by an amino-modified silicone oil.

Example 3

An experiment was carried out in the same manner as Example 1 above except that the amino-modified silicone oil shown in Table 2 was used. The result is shown in FIG. 5.

[Table 2]

| Component name | Product name | Viscosity ($mm^2/s$) |
|---|---|---|
| Aminoethylaminopropylmethylsiloxane dimethylsiloxane copolymer | KF-8004 | 800 |
| Aminoethylaminopropylmethylsiloxane dimethylsiloxane copolymer | KF-867S | 1300 |

Like the result shown in FIG. 5, even in the case of a separate amino-modified silicone oil (product names of "KF-8004" and "KF-867S"), it was shown that the emulsifying action is exhibited by sodium dodecyl sulfate immediately after mixing, but the emulsified state was not maintained. On the other hand, it was clear that the emulsified state is relatively stably maintained by sodium surfactin.

Example 4

Furthermore, an experiment was carried out in the same manner as Example 3 above except that the concentration of the surfactant is reduced to 0.5 mM with respect to the entire mixture. The result is shown in FIG. 6.

Like the result shown in FIG. 6, in the case of the amino-modified silicone oil with product name of "KF-867S", the emulsified state cannot be also maintained by sodium dodecyl sulfate; on the other hand, although the emulsifying effect by sodium surfactin was reduced, the emulsified state was maintained. Furthermore, also in the case of the amino-modified silicone oil with product name of "KF-8004", the emulsified state was relatively maintained by sodium surfactin.

According to the experiments in the above, it was proved that sodium surfactin not only can suitably emulsify the mixture of an aqueous solvent and an amino-modified silicone oil but also can stably maintain the emulsified state.

## Claims

1. An emulsified composition comprising an amino-modified silicone oil, an aqueous solvent, and a surfactin represented by the formula (I) below:

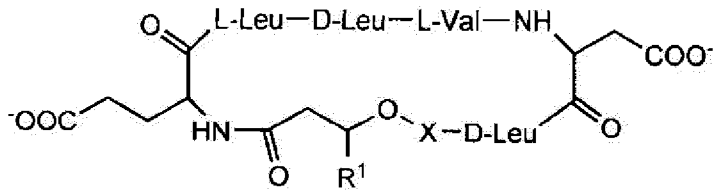

(I)

in the formula,

X represents an amino acid residue selected from the group consisting of a leucine residue, an isoleucine residue, and a valine residue; and
$R^1$ represents a $C_{9-18}$ alkyl group.

2. The emulsified composition according to claim 1, further comprising an oily component.

3. The emulsified composition according to claim 1 or 2, wherein a concentration of the surfactin is 0.1 mM or more and 50 mM or less with respect to the entire emulsified composition.

4. A cosmetic comprising the emulsified composition according to any one of claims 1 to 3.

FIG. 1

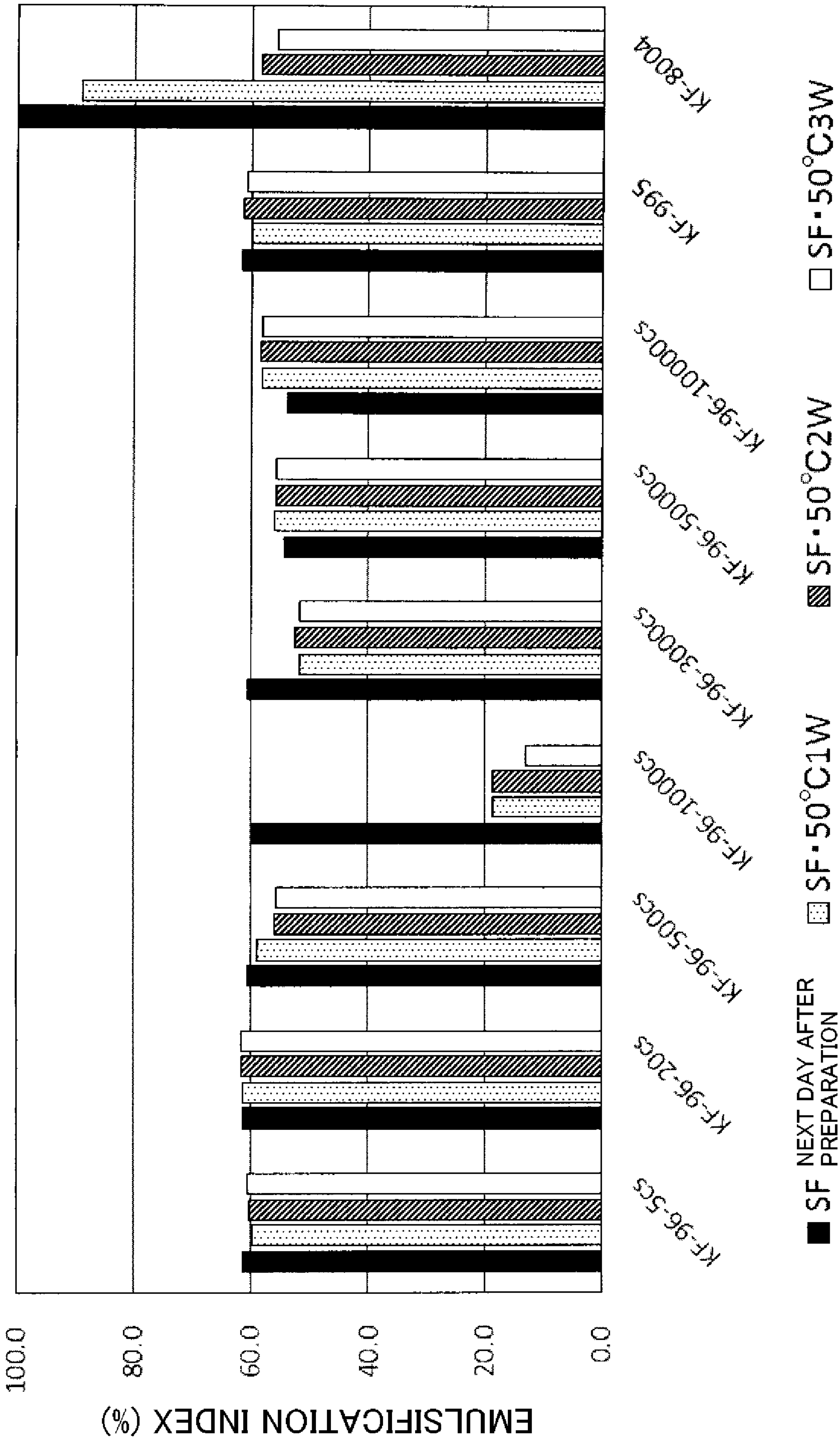

EMULSIFICATION INDEX (%)
100.0
80.0
60.0
40.0
20.0
0.0
KF-96-5cs
KF-96-20cs
KF-96-500cs
KF-96-1000cs
KF-96-3000cs
KF-96-5000cs
KF-96-10000cs
KF-995
KF-8004
SF NEXT DAY AFTER PREPARATION
SF·50°C1W
SF·50°C2W
SF·50°C3W

FIG. 2

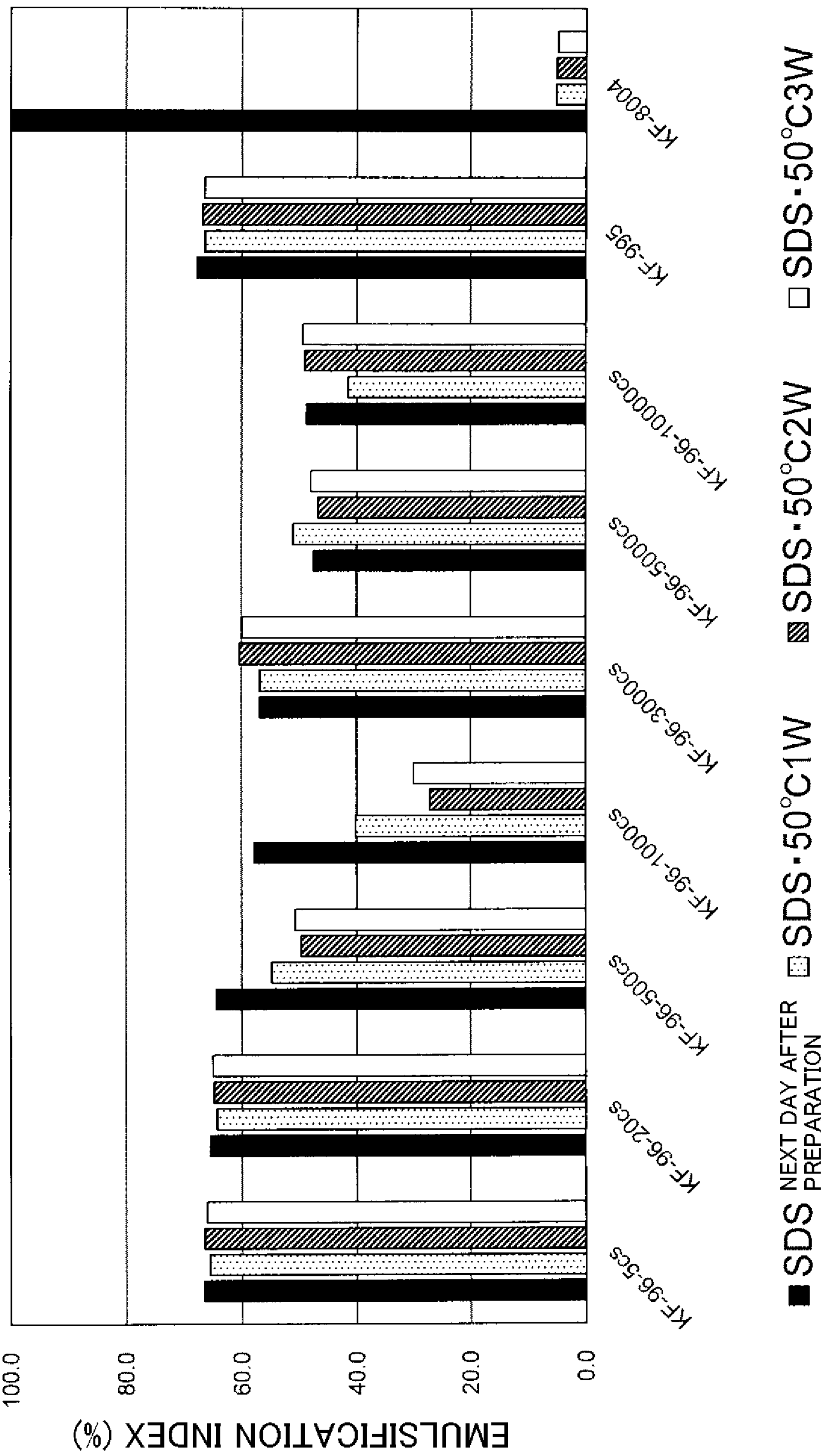
EMULSIFICATION INDEX (%)
100.0
80.0
60.0
40.0
20.0
0.0
KF-96-5cs
KF-96-20cs
KF-96-500cs
KF-96-1000cs
KF-96-3000cs
KF-96-5000cs
KF-96-10000cs
KF-995
KF-8004
SDS NEXT DAY AFTER PREPARATION
SDS·50°C1W
SDS·50°C2W
SDS·50°C3W

FIG. 3

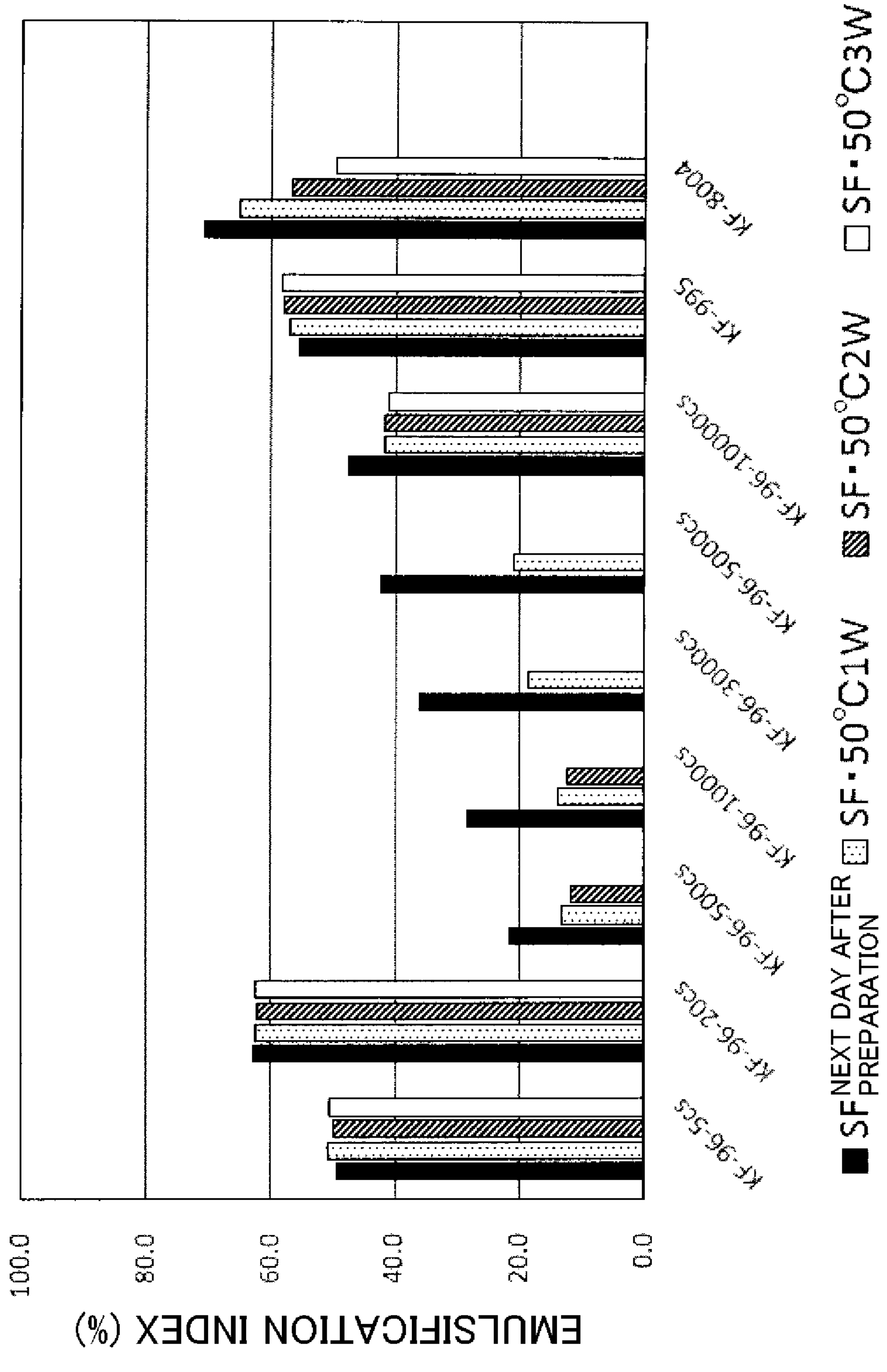
EMULSIFICATION INDEX (%)
100.0
80.0
60.0
40.0
20.0
0.0
KF-96-5cs
KF-96-20cs
KF-96-500cs
KF-96-1000cs
KF-96-3000cs
KF-96-5000cs
KF-96-10000cs
KF-995
KF-8004
SF NEXT DAY AFTER PREPARATION
SF·50°C1W
SF·50°C2W
SF·50°C3W

FIG. 4

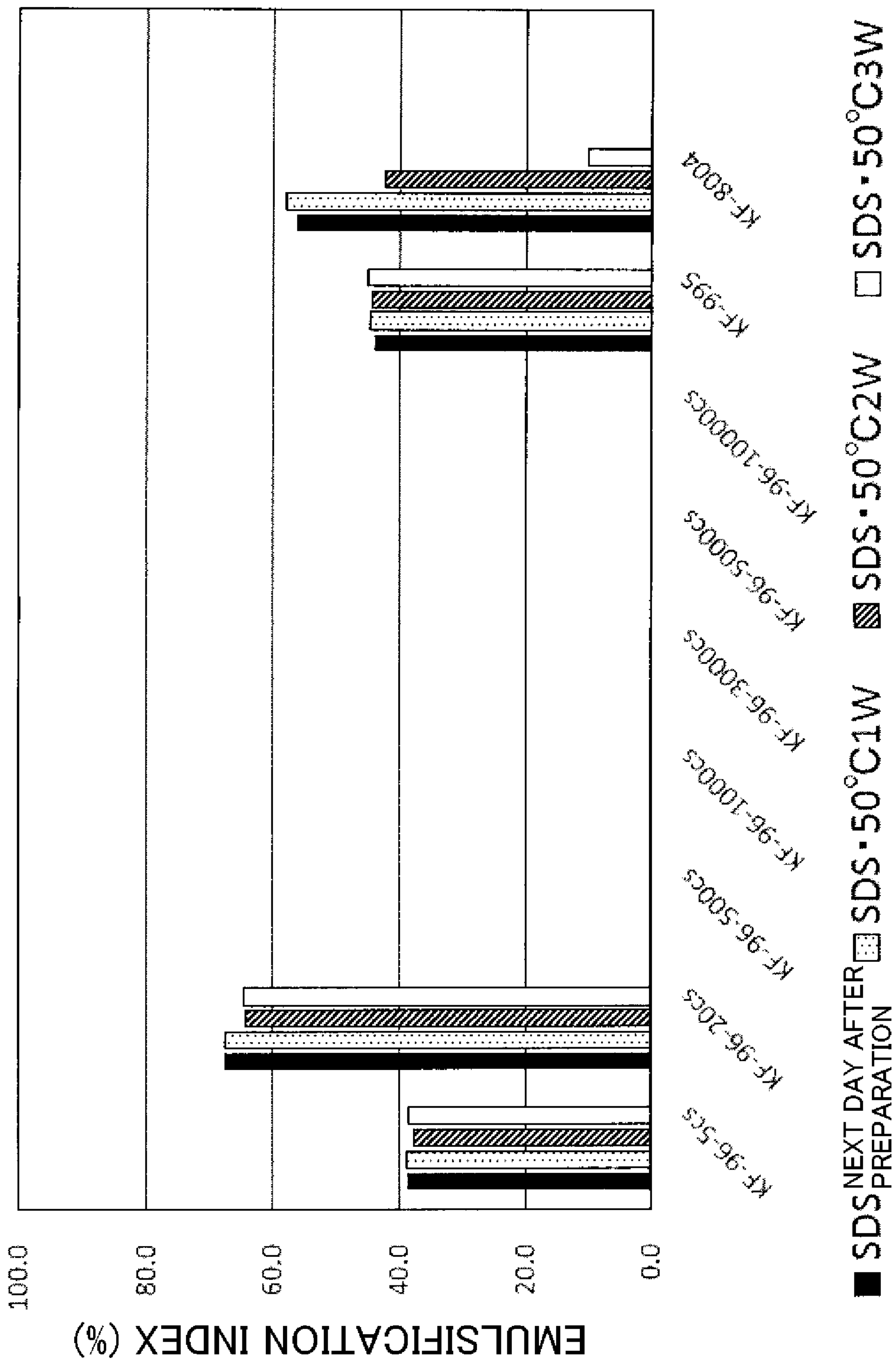
EMULSIFICATION INDEX (%)
100.0
80.0
60.0
40.0
20.0
0.0
KF-96-5cs
KF-96-20cs
KF-96-500cs
KF-96-1000cs
KF-96-3000cs
KF-96-5000cs
KF-96-10000cs
KF-995
KF-8004
SDS NEXT DAY AFTER PREPARATION
SDS·50°C1W
SDS·50°C2W
SDS·50°C3W

FIG. 5

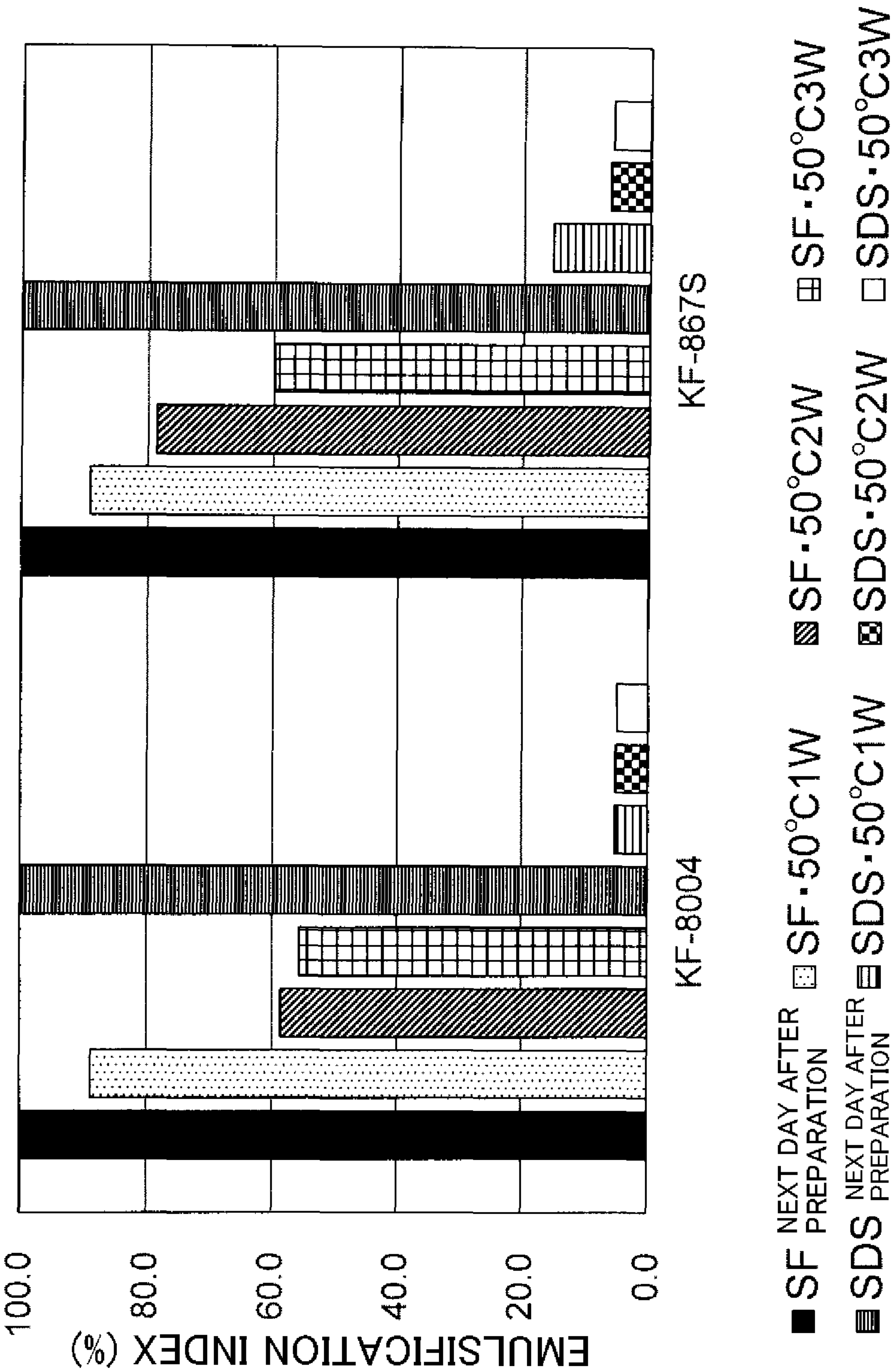
EMULSIFICATION INDEX (%)
100.0
80.0
60.0
40.0
20.0
0.0
KF-8004
KF-867S
SF NEXT DAY AFTER PREPARATION
SF・50°C1W
SF・50°C2W
SF・50°C3W
SDS NEXT DAY AFTER PREPARATION
SDS・50°C1W
SDS・50°C2W
SDS・50°C3W

FIG. 6

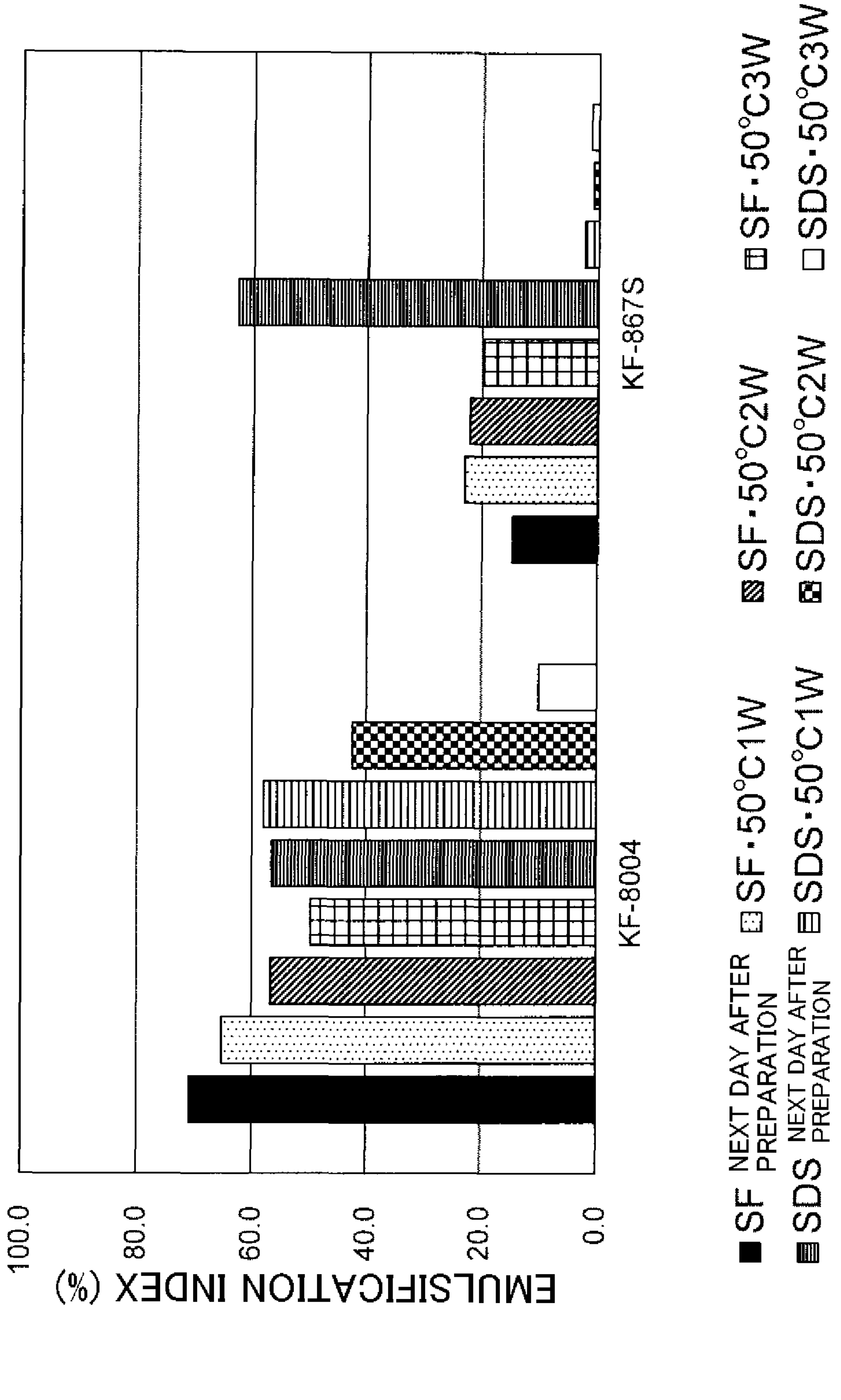
EMULSIFICATION INDEX (%)
100.0
80.0
60.0
40.0
20.0
0.0
KF-8004
KF-867S
SF NEXT DAY AFTER PREPARATION
SF·50°C1W
SF·50°C2W
SF·50°C3W
SDS NEXT DAY AFTER PREPARATION
SDS·50°C1W
SDS·50°C2W
SDS·50°C3W

**INTERNATIONAL SEARCH REPORT**

International application No. PCT/JP2017/024552

A. CLASSIFICATION OF SUBJECT MATTER
*A61K8/64*(2006.01)i, *A61K8/06*(2006.01)i, *A61K8/898*(2006.01)i, *A61Q19/00*(2006.01)i, *B01F17/30*(2006.01)i

According to International Patent Classification (IPC) or to both national classification and IPC

B. FIELDS SEARCHED

Minimum documentation searched (classification system followed by classification symbols)
A61K8/64, A61K8/06, A61K8/898, A61Q19/00, B01F17/30

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2011-26281 A (Toyobo Co., Ltd.), 10 February 2011 (10.02.2011), entire text (particularly, claims; paragraph [0009]; blending examples) (Family: none) | 1-4 |
| A | JP 58-63750 A (Asahi Denka Co., Ltd.), 15 April 1983 (15.04.1983), entire text (particularly, claims; page 1, lower right column, line 11 to page 2, lower left column, line 13; examples) (Family: none) | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 August 2017 (04.08.17) | 22 August 2017 (22.08.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/024552

C (Continuation). DOCUMENTS CONSIDERED TO BE RELEVANT

| Category* | Citation of document, with indication, where appropriate, of the relevant passages | Relevant to claim No. |
|---|---|---|
| A | WO 2015/013247 A1 (THE PROCTER & GAMBLE CO.), 29 January 2015 (29.01.2015), entire text & JP 2016-534177 A & US 2015/0030643 A1 & EP 3024430 A1 & CN 105407859 A | 1-4 |
| A | JP 2009-503019 A (Coty Deutschland GmbH), 29 January 2009 (29.01.2009), entire text & US 2008/0317686 A1 & WO 2007/014908 A1 & EP 1909749 A1 & KR 10-2008-0034169 A | 1-4 |
| A | JP 2005-306863 A (Showa Denko Kabushiki Kaisha), 04 November 2005 (04.11.2005), entire text & US 2008/0311234 A1 & WO 2005/089708 A1 & EP 1742606 A1 & KR 10-2006-0130215 A & TW 200533378 A | 1-4 |
| A | JP 2006-265187 A (Showa Denko Kabushiki Kaisha), 05 October 2006 (05.10.2006), entire text & US 2008/0085251 A1 & WO 2006/068276 A1 & EP 1827370 A & KR 10-2007-0089248 A & CN 101083975 A & CN 101669887 A & TW 200637595 A | 1-4 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011026280 A
- JP 2011026281 A
- JP 2003176211 A